# FASCICULE DE BREVET EUROPEEN

(11) **EP 3 958 995 B1**
(45) Date de publication et mention de la délivrance du brevet: **05.04.2023**
(21) Numéro de dépôt: 20717918.5
(22) Date de dépôt: 17.04.2020
(51) Int. Cl.: B01D 15/18, C07C 7/12, C07C 15/08

(54) **DISPOSITIF DE DISTRIBUTION PAR PANNEAUX MÉRIDIENS AVEC SÉPARATION DES RÉSEAUX DE COLLECTE**
VORRICHTUNG ZUR VERTEILUNG DURCH MERIDIONALE PANEELE MIT TRENNUNG VON SAMMELNETZEN
DEVICE FOR DISTRIBUTION THROUGH MERIDIONAL PANELS WITH SEPARATION OF COLLECTION NETWORKS

(30) Priorité: 25.04.2019 FR 1904396
(43) Date de publication de la demande: 02.03.2022
(73) Titulaire: IFP Energies nouvelles, 92852 Rueil-Malmaison Cedex (FR)
(72) Inventeur: AUGIER, Frederic, 92852 RUEIL-MALMAISON CEDEX (FR); ROYON-LEBEAUD, Aude, 92852 RUEIL-MALMAISON CEDEX (FR); FOURATI, Manel, 92852 RUEIL-MALMAISON CEDEX (FR)
(86) Numéro de dépôt international: PCT/EP2020/060823
(87) Numéro de publication internationale: WO 2020/216685

(56) Documents cités:
- EP-A1- 0 821 988
- WO-A1-2019/081311
- FR-A1- 2 782 657
- FR-A1- 2 930 454

## Description

### Domaine technique

La présente invention concerne un dispositif d'introduction et de collecte des fluides dans le procédé de séparation des xylènes en lit mobile simulé, et les unités mettant en oeuvre ledit procédé, plus particulièrement les unités de grands diamètres (D > 1m), et possédant de nombreux étages de séparation avec injection ou soutirage de produits entre deux étages.

### Technique antérieure

Les technologies actuelles de séparation en lit mobile simulé (parfois en abrégé LMS dans la suite du texte) utilisent des unités qui comportent un certain nombre de points communs :
- une succession de lits d'adsorbant au sein desquels s'écoule un fluide selon un débit de « tourne en rond » ; ce débit de tourne en rond représente en général plusieurs fois le débit de charge entrant (environ entre 1,5 et 6 fois) ; on appelle « tourne en rond » dans la terminologie anglo-saxonne « pump around »,
- des systèmes d'injection de la charge et du solvant, et de soutirage des effluents appelés extrait et raffinat,
- des systèmes de collecte et redistribution pour passer d'un lit au lit suivant.

Dans les procédés de séparation par adsorption en lit mobile simulé on dispose généralement d'une pluralité de lits localisés dans une ou deux colonnes d'adsorption. Entre chaque lit se situent des panneaux distributeurs-mélangeurs-extracteurs ou « DME » alimentés par des lignes qui présentent le plus souvent la forme d'« araignées de distribution/ extraction ». Chaque panneau DME situé entre deux lits consécutifs est relié à l'extérieur au moyen d'une ou deux lignes ou réseaux aboutissant à une vanne mettant en communication successivement chacun des lits avec chacun des flux entrant dans ou sortant de la section d'adsorption. Cette opération est réalisée de manière séquentielle, et le temps au bout duquel on revient au lit de départ s'appelle le temps de cycle.

Par exemple, le brevet US2985589 montre clairement que chacun des réseaux d'injection ou soutirage est relié par une seule ligne à une vanne qui connecte successivement la charge, l'extrait, le solvant puis le raffinat. Cette manière de procéder présente l'inconvénient de diminuer considérablement les performances du procédé puisque chaque flux se trouve ainsi contaminé par le contenu de la ligne commune. Il est donc indispensable de mettre en place un système de rinçage.

Plusieurs brevets expliquent comment mettre en oeuvre ces rinçages, notamment les brevets FR 2930454, FR 2782657, FR2751888, FR2772634, FR2870751.

Les rinçages s'avèrent en général coûteux en matière d'investissement (e.g. vannes et lignes supplémentaires), aussi bien qu'en matière de coût opératoire (rendement, productivité).

Les « araignées de distribution/extraction » constituent des obstacles au sein du lit d'adsorbant qui perturbent l'écoulement dans le lit. Les obstacles dans le lit ont un coût opératoire quant à la productivité et le rendement. Le brevet WO09133254 montre comment minimiser l'impact des obstacles sur l'hydrodynamique dans le lit. L'article Augier et al. 2008 (Séparation and Purification Technology 63, pp466-474) évalue le coût des obstacles.

### Résumé de l'invention

Selon un premier aspect, la présente invention concerne un dispositif de distribution et de collecte pour une unité de séparation en lit mobile simulé de diamètre supérieur à 1 mètre, le dispositif comprenant au moins une colonne divisée en une pluralité de lits d'adsorbant et des plateaux supportant les lits d'adsorbant, dans lequel : au moins un plateau disposé entre un premier lit d'adsorbant et un deuxième lit d'adsorbant (sous-jacent), est divisé en panneaux méridiens parallèles entre eux et contigus pour couvrir la section dudit plateau ; et chaque panneau méridien comprend un canal de collecte adapté pour le soutirage d'un fluide du premier lit d'adsorbant, un canal de distribution adapté pour la distribution du fluide vers le deuxième lit d'adsorbant, et une plaque de séparation pour séparer le canal de collecte et le canal de distribution, le dispositif comprenant en outre une pluralité de conduites périphériques extérieures à la colonne, chaque conduite périphérique étant adaptée pour relier exclusivement un canal de collecte d'un panneau méridien au canal de distribution dudit panneau méridien.

Avantageusement, le dispositif selon la présente invention permet : d'assurer une collecte globale du flux de « tourne en rond » à chaque panneau pour s'affranchir des rinçages ; de minimiser les obstacles au sein du lit ; et de respecter un temps de séjour à peu près égal pour les fluides dans le réseau externe à la colonne ainsi que dans la colonne.

Selon un ou plusieurs modes de réalisation, le canal de distribution et le canal de collecte ont des hauteurs variant linéairement sur la longueur du panneau méridien, telles que : la somme des hauteurs du canal de distribution et du canal de collecte prises en tout point de la longueur du panneau méridien est sensiblement constante ; et la vitesse d'entrée dans le deuxième lit d'adsorbant de particules ou molécules du fluide reste sensiblement constante depuis le point d'entrée du canal de distribution sur toute la longueur du panneau méridien.

Selon un ou plusieurs modes de réalisation, le plateau est divisé entre 2 et 12 panneaux méridiens.

Selon un ou plusieurs modes de réalisation, les conduites périphériques comprennent au moins un point d'injection ou de soutirage pour injecter une charge ou un solvant dans la colonne ou soutirer un raffinat ou un d'extrait de la colonne.

Selon un ou plusieurs modes de réalisation, le dispositif comprend une pluralité de grilles supérieures et de grilles inférieures, les panneaux méridiens du plateau étant disposés entre la grille inférieure du premier lit d'adsorbant et la grille supérieure du deuxième lit d'adsorbant.

Selon un ou plusieurs modes de réalisation, les longueurs des conduites périphériques connectées au plateau sont prédéterminées pour permettre des temps de séjour identiques des particules ou molécules du fluide traversant lesdites conduites périphériques.

Selon un ou plusieurs modes de réalisation, chaque conduite périphérique suit sensiblement le périmètre de la colonne à partir du canal de collecte jusqu'au canal de distribution.

Selon un ou plusieurs modes de réalisation, les conduites périphériques sont essentiellement horizontales et sont situées en périphérie du plateau auquel les conduites périphériques sont connectées.

Selon un ou plusieurs modes de réalisation, au moins une conduite périphérique comprend au moins une partie verticale.

Selon un ou plusieurs modes de réalisation, le canal de collecte comprend une pluralité de points de sortie et le canal de distribution comprend une pluralité de points d'entrée, la pluralité de points de sortie étant reliée à la pluralité de points d'entrée par une conduite périphérique unique.

Selon un deuxième aspect, la présente invention concerne un procédé utilisant le dispositif selon le premier aspect, comprenant les étapes suivantes : soutirer un fluide d'un premier lit d'adsorbant par le canal de collecte d'un panneau méridien d'un plateau ; envoyer le fluide du canal de collecte exclusivement vers une conduite périphérique ; envoyer le fluide de la conduite périphérique exclusivement vers le canal de distribution dudit panneau méridien ; distribuer le fluide du canal de distribution dans un deuxième lit d'adsorbant.

Selon un ou plusieurs modes de réalisation, le procédé est utilisé pour la séparation des xylènes en lit mobile simulé fonctionnant avec un nombre de lits compris entre 4 et 24.

Selon un ou plusieurs modes de réalisation, le procédé comprenant en outre l'étape suivante : commander les débits des canaux de distribution du plateau pour alimenter en fluide le deuxième lit d'adsorbant avec des vitesses sensiblement égales.

Selon un ou plusieurs modes de réalisation, la vitesse maximale débitante du fluide dans les conduites périphériques est inférieure ou égale à 6 m/s.

Selon un ou plusieurs modes de réalisation, les conduites périphériques fonctionnent à sensiblement iso temps de séjour.

D'autres caractéristiques et avantages de l'invention des aspects précités, apparaîtront à la lecture de la description ci-après et d'exemples non limitatifs de réalisations, en se référant aux figures annexées et décrites ci-après.

### Liste des figures

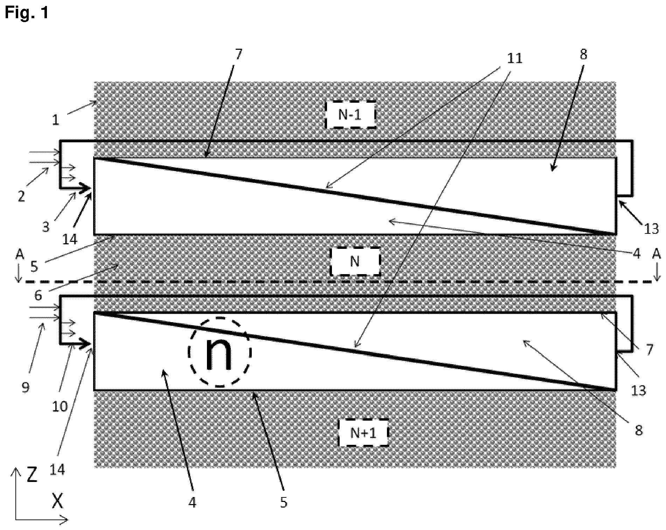

La figure 1 montre une vue en coupe de 3 lits d'adsorbants 6 successifs, notés N-1, N et N+1 de haut en bas et un plateau n disposé entre les lits d'adsorbants N et N+1. Elle permet de bien visualiser le canal de distribution 4 et le canal de collecte 8, symétrique l'un de l'autre et séparés par la paroi 11, avec notamment le passage du fluide par le conduite périphérique 10 provenant du lit N depuis le point de sortie 13 du canal de collecte 8 jusqu'au point d'entrée 14 du canal de distribution 4 alimentant le lit N+1.

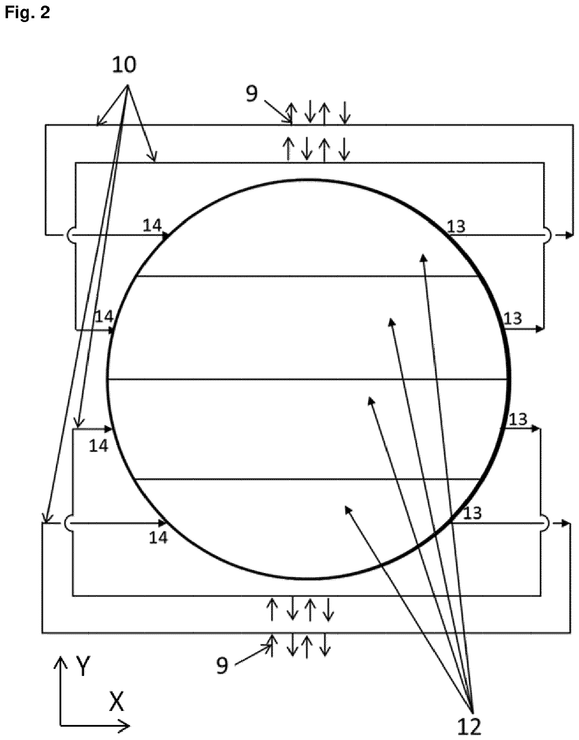

La figure 2 correspond à une vue de dessus de la colonne d'après une coupe selon la ligne A-A de la figure 1. Elle permet de visualiser un plateau n et sa division en panneaux méridiens ainsi que la collecte des fluides par les points de sortie 13 des canaux de collecte 8 jusqu'aux points d'entrée 14 des canaux de distribution 4. Les collectes par les conduites périphériques 10 restent séparées les unes des autres. Chaque conduite périphérique 10 relie le point de sortie 13 d'un canal de collecte 8 d'un panneau méridien au canal de distribution 4 du même panneau méridien.

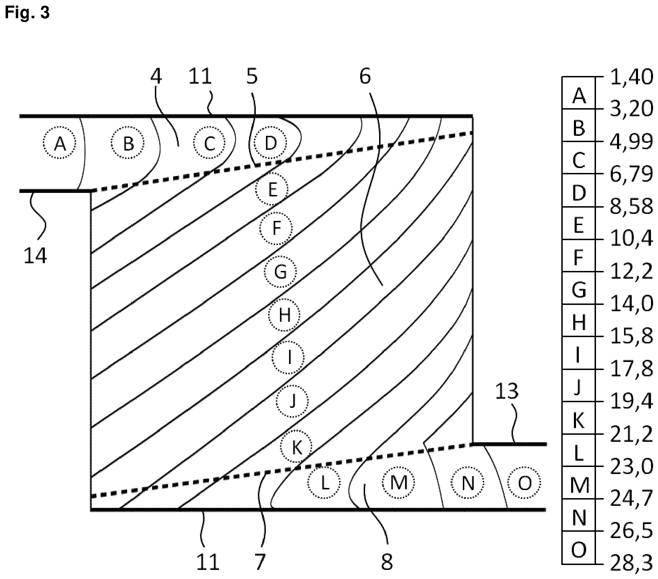

La figure 3 est une visualisation résultant d'une simulation numérique. Il s'agit d'une vue en coupe, de haut en bas, d'un canal de distribution 4 d'un lit d'adsorbant N noté 6, du lit d'adsorbant N et du canal de collecte 8 du lit d'adsorbant N, selon un plan de coupe identique à celui de la figure 1. Le point d'entrée 14 dans le canal de distribution 4 se fait par le bord en haut à gauche. Le lit d'adsorbant N est la zone comprise entre les deux grilles 5 et 7 schématisées par des lignes pointillées. Le point de sortie 13 du canal de collecte 8 se fait par le bord en bas à droite. Les zones A à O se réfèrent à l'âge interne moyen du fluide dans le lit en secondes.

### Description des modes de réalisation

la présente invention peut se définir comme un dispositif de distribution et de collecte pour les unités de séparation en lit mobile simulé de diamètre supérieur à 1 mètre, préférentiellement supérieur à 4 mètres, très préférentiellement supérieur à 7 mètres.

Le dispositif comprend au moins une colonne de séparation divisé en N lits d'adsorbant supportés par n plateaux (ou zones inter-lits), chaque plateau étant lui-même divisé en panneaux méridiens, c'est-à-dire en panneaux (ou compartiments) parallèles entre eux et contigus de manière à assurer une couverture complète de la section horizontale du plateau.

Préférablement, le nombre de lits d'adsorbant N et le nombre de panneaux n sont identiques et sont compris entre 4 et 24, et préférentiellement compris entre 8 et 12. Selon un ou plusieurs modes de réalisation, chaque plateau est divisé en entre 2 et 12 panneaux méridiens, préférentiellement entre 4 et 8 panneaux méridiens.

Chaque panneau méridien comprend un canal de collecte et un canal de distribution séparés entre eux par une plaque de séparation.

Le soutirage d'un fluide d'un lit d'adsorbant N est effectué par le canal de collecte d'un panneau méridien et la distribution dudit fluide vers le lit d'adsorbant N+1, situé (directement) en aval du lit d'adsorbant N, est effectué exclusivement par le canal de distribution du même panneau méridien. Ainsi, chaque plateau n comprend une pluralité de panneaux méridiens, chaque panneau méridien comprenant un canal de collecte du lit d'adsorbant N et un canal de distribution du lit d'adsorbant N+1.

En outre, le dispositif de distribution et de collecte selon l'invention comprend une pluralité de conduites périphériques extérieures à la colonne, chaque conduite périphérique reliant uniquement un canal de collecte d'un panneau méridien au canal de distribution dudit panneau méridien.

Selon un ou plusieurs modes de réalisation, ladite conduite périphérique permet en outre d'effectuer des injections de charge et de solvant de l'extérieur vers la colonne et des soutirages de raffinat et d'extrait de la colonne vers l'extérieur.

Avantageusement, le dispositif selon la présente invention est applicable aux unités en lit mobile simulé.

Avantageusement, le dispositif selon la présente invention permet d'assurer une collecte globale du flux de « tourne en rond » pour s'affranchir des rinçages. La collecte globale du flux de « tourne en rond » est un enjeu extrêmement important sur les unités en lit mobile simulé, puisqu'elle permet d'éliminer les rinçages.

Avantageusement, le dispositif selon la présente invention permet de minimiser les obstacles au sein du lit.

Avantageusement, le dispositif selon l'invention a la particularité de respecter un temps de séjour à peu près égal pour les fluides traversant le lit d'adsorbant N, les panneaux méridiens du plateau n et le lit d'adsorbant N+1.

La technologie décrite dans la présente invention utilise le principe de compensation des temps de séjour au sein des zones de collecte et de distribution pour minimiser les variances, c'est-à-dire les écarts de temps de séjour des fluides circulant dans l'unité en fonction du point de départ et du point d'arrivée desdits fluides. Les volumes inter-lits peuvent être minimisés en travaillant à iso-vitesse plutôt qu'à iso-hauteur de canal dans les zones de collecte et distribution. L'encombrement total de la colonne peut également être minimisé en empilant les lits. Il n'y a donc pas de gestion de volume inter-lit spécifique.

Selon un ou plusieurs modes de réalisation, le canal de distribution et le canal de collecte ont des hauteurs variant linéairement sur toute la longueur du panneau méridien, et sont telles que la somme des hauteurs du canal de distribution et du canal de collecte prise en tout point de la longueur du panneau méridien reste constante. Selon un ou plusieurs modes de réalisation, le canal de distribution et le canal de collecte ont des hauteurs telles que la vitesse d'entrée dans le lit d'adsorbant des particules fluides ou molécules reste la même depuis le point d'entrée du canal de distribution sur toute la longueur du panneau méridien.

Dans la présente demande, on appelle longueur du panneau méridien (identique à celle du canal de distribution et du canal de collecte) la dimension du panneau méridien correspondant à la distance traversant la partie médiane du panneau méridien selon sa dimension la plus grande (i.e., longueur sensiblement égale à la distance entre le point de sortie du canal de collecte et point d'entrée du canal de distribution d'un même panneau méridien). Dans la présente demande, on appelle largeur du panneau méridien (du canal de distribution et du canal de collecte) la dimension horizontale perpendiculaire à la longueur, et on appelle hauteur la dimension verticale perpendiculaire à la longueur. Plus précisément, la hauteur du canal de distribution décroit linéairement depuis le point d'entrée (extrémité du canal de distribution connectée à la conduite périphérique) jusqu'au niveau du point de sortie du canal de collecte (extrémité du canal de distribution opposée au point d'entrée), et la hauteur du canal de collecte décroit linéairement depuis le point de sortie (extrémité du canal de collecte connectée à la conduite périphérique) jusqu'au niveau du point d'entrée du canal de distribution (extrémité du canal de collecte opposée au point de sortie). A chaque abscisse x correspondant à un point courant de la longueur du panneau méridien, la somme des hauteurs du canal de distribution et du canal de collecte est constante.

En référence à la figure 1, le fluide provenant du lit d'adsorbant N-1, noté 1, est collecté dans la conduite périphérique 3 (via le point de sortie 13).

Les injections ou soutirages de la conduite périphérique 3 sont optionnellement réalisés par le point d'injection et de soutirage 2.

Le canal de distribution 4 est alimenté par la conduite périphérique 3 (via le point d'entrée 14) et assure un débit uniforme du fluide dans le lit N, noté 6 sur la figure 1, au travers d'une grille supérieure 5.

Le fluide traversant le lit N est collecté par le canal de collecte 8 au travers d'une grille inférieure 7.

Selon un ou plusieurs modes de réalisation, le lit d'adsorbant est délimité par une grille supérieure et une grille inférieure, les panneaux méridiens d'un plateau n étant disposés entre la grille inférieure du lit d'adsorbant N et la grille supérieure du lit d'adsorbant N+1.

Le canal de collecte 8 alimente la conduite périphérique 10 (via le point de sortie 13), pour être réinjecté au lit N+1 (via le point entrée 14), situé immédiatement au-dessous du lit N, au travers d'une grille supérieure 5.

Selon un ou plusieurs modes de réalisation, la conduite périphérique 10 comprend au moins un point d'injection ou de soutirage 9, externe à la colonne, pour injecter une charge ou un solvant dans la colonne ou soutirer un raffinat ou un d'extrait de la colonne.

Les plaques de séparation 11 séparent le canal de collecte 8 du canal de distribution 4. La planéité de la plaque de séparation 11 peut être assurée par tout moyen connu de l'homme du métier. On pourra par exemple rendre la plaque de séparation 11 solidaire des grilles inférieure et supérieure 5 et 7.

On peut également utiliser des tirants s'étendant sur toute la largeur du panneau méridien, rapportés sur des poutres ou plaques qui viennent délimiter lesdits panneaux méridiens sur la hauteur des canaux de collecte et distribution.

La hauteur au point d'entrée du canal de distribution 4 est définie par une vitesse débitante maximale admissible de manière à ne pas déséquilibrer l'alimentation du lit. Selon un ou plusieurs modes de réalisation, la vitesse débitante maximale admissible est comprise entre 0,1 et 5 m/s, idéalement entre 0,5 et 2,5 m/s.

La section de canal de distribution 4 diminue linéairement pour garantir une vitesse quasiment uniforme sur toute la longueur du canal, e.g. égale à une vitesse débitante maximale. Cette constance de la vitesse provient du fait que le débit de fluide est toujours proportionnel à la section entrante, ceci sur chaque section entrante du canal. Le profil de hauteur du canal de distribution est donc linéaire pour assurer cette proportionnalité. Pareillement, la section de canal de collecte 8 augmente linéairement, pour garantir une vitesse quasiment uniforme sur toute la longueur du canal, e.g. égale à une vitesse débitante maximale. Dans la présente demande, les termes « section de canal de distribution » et « section de canal de collecte » correspondent aux coupes verticales desdits canaux parallèles à la hauteur (axe Z) et perpendiculaires à la longueur du panneau méridien (axe X).

Les canaux de collecte 8 et distribution 4 sont complémentaires au sens où le canal de distribution 4 situé immédiatement au-dessus du lit N est associé au canal de collecte 8 situé immédiatement au-dessous dudit lit N. Le flux quittant le canal de collecte 8 est ensuite dirigé vers le canal de distribution du lit N+1 au moyen d'un conduite périphérique 10 dédiée, telle que montrée sur la figure 2.

La figure 2 montre un plateau n supportant un lit d'adsorbant N et divisé en panneaux méridiens 12, les panneaux méridiens 12 sont parallèles les uns aux autres et contigus, de manière à assurer une couverture totale de la section du plateau n (i.e., plan XY). La figure 2 montre également la collecte des fluides par le point de sortie 13 du canal de collecte 8 jusqu'au point d'entrée 14 du canal de distribution 4.

Les collectes par les conduites périphériques 10 restent séparées les unes des autres. Chaque conduite périphérique 10 relie exclusivement le point de sortie 13 d'un canal de collecte 8 d'un panneau méridien 12 au point d'entrée 14 du canal de distribution 4 du même panneau méridien 12. Le terme « relier exclusivement » signifie que chaque conduite périphérique 10 connectée à un canal de collecte 8 et un canal de distribution 4 d'un panneau méridien donné, n'est pas connectée à un canal de collecte 8 et/ou un canal de distribution 4 d'un autre panneau méridien. Il est entendu qu'une conduite périphérique 10 peut comprendre des points d'injection et de soutirage 9 externes à la colonne pour notamment injecter une charge ou un solvant ou soutirer un raffinat ou un extrait.

Selon un ou plusieurs modes de réalisation, les plateaux n sont préférentiellement organisés en panneaux méridiens de hauteur constante.

Selon un ou plusieurs modes de réalisation, les débits des canaux de distribution 4 sont ajustés pour avoir la même vitesse au sein du lit d'adsorbant 6.

Selon un ou plusieurs modes de réalisation, chaque conduite périphérique 10 suit grossièrement le périmètre cylindrique de la colonne pour connecter le point de sortie 13 d'un canal de collecte 8 d'un panneau méridien 12 au point d'entrée 14 du canal de distribution 4 du panneau méridien 12.

Selon un ou plusieurs modes de réalisation, les conduites périphériques 10 sont dimensionnées pour que la vitesse maximale débitante ne dépasse pas une certaine vitesse maximale, typiquement inférieure ou égale à 6 m/s et générale comprise entre 4 et 6 m/s (par exemple, pour des raisons de vibration). Selon un ou plusieurs modes de réalisation, la vitesse maximale débitante du fluide dans les conduites périphériques (10) est comprise entre 3 et 5 m/s.

On parle de temps de séjour « réseau » pour désigner le temps de séjour d'une particule ou molécule du fluide dans une conduite périphérique 10, depuis le point de sortie 13 du canal de collecte 8 jusqu'au point d'entrée 14 du canal de distribution 4.

Le réseau externe d'un plateau n est formé par le nombre de conduites périphériques 10 égal au nombre de panneaux méridiens 12 du plateau n. Le réseau externe permet d'assurer le même temps de séjour pour toutes les particules ou molécules du fluide entre le point de collecte et le point de distribution pour un plateau n.

Selon un ou plusieurs modes de réalisation, les conduites périphériques 10 d'un réseau externe sont conçues pour fonctionner à iso temps de séjour.

On peut distinguer :
- le temps de séjour réseau côté collecte, qui est le temps de séjour de la particule ou molécule du fluide depuis son point de sortie 13 de la colonne depuis n'importe quel panneau méridien, jusqu'aux points d'injection ou de soutirage 9,
- le temps de séjour réseau côté distribution qui est le temps de séjour de la particule ou molécule du fluide depuis le point d'injection ou soutirage 9 jusqu'à son point d'entrée 14 dans la colonne vers n'importe quel panneau méridien.

Selon un ou plusieurs modes de réalisation, le temps de séjour d'un réseau externe est identique au sein des conduites périphériques 10 du point de sortie 13 au point d'injection ou de soutirage 9, et le temps de séjour d'un réseau externe est identique au sein des conduites périphériques 10 du point d'injection ou de soutirage 9 au point d'entrée 14.

Selon un ou plusieurs modes de réalisation, les longueurs des conduites périphériques 10 d'un réseau externe sont variables pour permettre un temps de séjour identique des particules ou molécules du fluide les traversant. Selon un ou plusieurs modes de réalisation, les conduites périphériques 10 sont essentiellement horizontales et sont situées dans un même niveau (une même hauteur) de la colonne, optionnellement avec des longueurs différentes. Selon un ou plusieurs modes de réalisation, les conduites périphériques 10 comprennent des parties verticales permettant d'obtenir les longueurs de conduites voulues. Avantageusement, le dispositif de distribution et de collecte gagne en compacité.

Selon un ou plusieurs modes de réalisation, les points d'entrée 14 des panneaux méridiens 12 sont réalisées par l'intermédiaire de 1 à 6 points d'entrée. Selon un ou plusieurs modes de réalisation, les points de sortie 13 des panneaux méridiens 12 sont réalisées par l'intermédiaire de 1 à 6 points de sortie.

La présente invention porte également sur un procédé utilisant le dispositif de distribution et de collecte selon le premier aspect, procédé dans lequel une fraction d'effluent d'un lit d'adsorbant N entre dans un canal de collecte d'un panneau méridien d'un plateau n, puis dans une conduite périphérique reliant uniquement le canal de collecte au canal de distribution dudit panneau méridien, puis dans ledit canal de distribution, et puis dans le lit d'adsorbant N+1. Le procédé selon l'invention est particulièrement adapté pour la séparation des xylènes en lit mobile simulé fonctionnant avec un nombre de lits compris entre 4 et 24, et préférentiellement compris entre 8 et 12.

### Exemple

Une unité d'adsorption en lit mobile simulé (ou adsorbeur) de 10 mètres de diamètre comprend des plateaux divisés en 6 panneaux méridiens, et est alimenté suivant le principe de l'invention présenté sur la figure 1. Chaque lit a une hauteur de 0,77 mètre.

Des simulations réalisées à l'aide du logiciel de mécanique des fluides numérique FLUENT18.0 montrent que le principe de la compensation des temps de séjour entre le point d'entrée 14 et le point de sortie 13 fonctionne correctement. Ce fonctionnement satisfaisant est illustré par la figure 3.

La figure 3 est une visualisation résultant de cette simulation numérique. Il s'agit d'une vue en coupe d'un lit d'adsorbant N disposé entre un canal de distribution 4 et un canal de collecte 8, selon un plan de coupe similaire à la figure 1. Le point d'entrée 14 du canal de distribution 4 se trouve au bord en haut à gauche. Le lit d'adsorbant 6 est la zone comprise entre la grille supérieure 5 et la grille inférieure 7 schématisées par des lignes pointillées. Le point de sortie 13 du canal de collecte 8 se trouve au bord en bas à droite.

Le canal de distribution 4 du lit d'adsorbant 6 a une hauteur de 19 cm au point le plus haut, c'est-à-dire au niveau du point d'entrée 14 du fluide. La hauteur du canal décroit ensuite de façon linéaire en fonction de la distance avec le point d'entrée 14. Dans cet exemple, le canal de collecte 8 du lit d'adsorbant 6 est strictement symétrique au canal de distribution 4. Il a une hauteur croissante depuis le côté gauche jusqu'au point de sortie 13 disposé du côté droit du canal de collecte 8.

La figure 3 montre en outre une cartographie des temps de séjour (ou âge moyen interne, c'est-à-dire du temps s'étant écoulé depuis le point d'entrée 14 jusqu'au point de sortie 13 d'une particule ou molécule prédéterminée du fluide) en tout point du système global comprenant le canal de distribution 4, le lit d'adsorbant 6 et le canal de collecte 8.

La grille des zones A à O représentée à droite sur la figure 3 se réfère à l'âge interne moyen du fluide dans le lit en secondes, et indique les variations du temps de séjour global entre environ 1,40 secondes représentées par la zone A et environ 28,3 secondes représenté par la zone O. Ainsi une particule ou molécule du fluide qui vient d'entrer dans le canal de distribution 4 au point d'entrée 14 a un temps de séjour proche de 1 seconde. Quand une particule ou molécule du fluide quitte le canal de collecte 8 en bas à droite au point de sortie 13, son temps de séjour est de l'ordre de 28 secondes.

Les iso-lignes de temps de séjour ne sont pas horizontales dans le lit. Sur une même côte verticale au sein du lit, la particule ou molécule du fluide qui est rentrée à gauche dans le lit, près de l'entrée dans la colonne a un temps de séjour très court dans la zone de distribution, donc un temps de séjour global toujours plus élevé que la particule qui est rentrée dans le lit à droite qui a un temps de séjour dans le canal de distribution plus long. En revanche, le temps de séjour dans le lit de toutes les particules ou molécules du fluide est identique.

Les simulations montrent qu'en sortie on a récupéré les écarts de temps de séjour générés dans le canal de distribution 4 en les compensant par les variations de temps de séjour dans le canal de collecte 8. Le profil de temps de séjour se retrouve quasiment perpendiculaire au sens d'écoulement dans le canal de sortie comme dans le canal d'entrée.

Les calculs montrent une dispersion très faible de l'ordre de 2 s² ce qui équivaut à une hauteur équivalente à un plateau théorique de l'ordre de 2 mm. Il s'agit là d'un excellent résultat en matière d'uniformité du temps de séjour.

Dans l'article Augier et al., 2008, on trouve des HETP (hauteur de plateau théorique) typiques d'une technologie d'une unité d'adsorption de l'ordre du centimètre.

On peut se reporter à la figure 9 p 473 de l'article cité qui représente la HETP pour différentes vitesses superficielles liquide au sein du lit. On retient la courbe qui correspond à deux configurations de technologies différentes en l'absence d'adsorption. Les valeurs estimées sont comprises entre 12 et 20 cm.

L'invention présente donc un gain d'un rapport 5 à 10 sur la dispersion imputable à l'hydrodynamique dont l'homme du métier sait qu'elle se répercute directement sur les performances du procédé.

## Revendications

1. Dispositif de distribution et de collecte pour une unité de séparation en lit mobile simulé de diamètre supérieur à 1 mètre, le dispositif comprenant au moins une colonne divisée en une pluralité de lits d'adsorbant (6) et des plateaux (n) supportant les lits d'adsorbant (6), dans lequel :
au moins un plateau (n) disposé entre un premier lit d'adsorbant (N) et un deuxième lit d'adsorbant (N+1), est divisé en panneaux méridiens (12) parallèles entre eux et contigus pour couvrir la section dudit plateau (n) ; et
chaque panneau méridien (12) comprend un canal de collecte (8) adapté pour le soutirage d'un fluide du premier lit d'adsorbant (N), un canal de distribution (4) adapté pour la distribution du fluide vers le deuxième lit d'adsorbant (N+1), et une plaque de séparation (11) pour séparer le canal de collecte (8) et le canal de distribution (4),
**caractérisé**
**en ce que** le dispositif comprend une pluralité de conduites périphériques (10) extérieures à la colonne, chaque conduite périphérique (10) étant adaptée pour relier exclusivement un canal de collecte (8) d'un panneau méridien au canal de distribution (4) dudit panneau méridien.

2. Dispositif de distribution et de collecte selon la revendication 1, dans lequel le canal de distribution (4) et le canal de collecte (8) ont des hauteurs variant linéairement sur la longueur du panneau méridien (12), telles que : la somme des hauteurs du canal de distribution (4) et du canal de collecte (8) prises en tout point de la longueur du panneau méridien (12) est sensiblement constante ; et la vitesse d'entrée dans le deuxième lit d'adsorbant (N+1) de particules ou molécules du fluide reste sensiblement constante depuis le point d'entrée du canal de distribution (4) sur toute la longueur du panneau méridien.

3. Dispositif de distribution et de collecte selon la revendication 1 ou la revendication 2, dans lequel le plateau (n) est divisé entre 2 et 12 panneaux méridiens (12).

4. Dispositif de distribution et de collecte selon l'une quelconque des revendications précédentes, dans lequel les conduites périphériques (10) comprennent au moins un point d'injection ou de soutirage (9) pour injecter une charge ou un solvant dans la colonne ou soutirer un raffinat ou un d'extrait de la colonne.

5. Dispositif de distribution et de collecte selon l'une quelconque des revendications précédentes, comprenant une pluralité de grilles supérieures (7) et de grilles inférieures (5), les panneaux méridiens (12) du plateau (n) étant disposés entre la grille inférieure (5) du premier lit d'adsorbant (N) et la grille supérieure du deuxième lit d'adsorbant (N+1).

6. Dispositif de distribution et de collecte selon l'une quelconque des revendications précédentes, dans lequel les longueurs des conduites périphériques (10) connectées au plateau (n) sont prédéterminées pour permettre des temps de séjour identiques des particules ou molécules du fluide traversant lesdites conduites périphériques (10).

7. Dispositif de distribution et de collecte selon l'une quelconque des revendications précédentes, dans lequel chaque conduite périphérique (10) suit sensiblement le périmètre de la colonne à partir du canal de collecte (8) jusqu'au canal de distribution (4).

8. Dispositif de distribution et de collecte selon l'une quelconque des revendications précédentes, dans lequel les conduites périphériques (10) sont essentiellement horizontales et sont situées en périphérie du plateau (n) auquel les conduites périphériques (10) sont connectées.

9. Dispositif de distribution et de collecte selon l'une quelconque des revendications précédentes, dans lequel au moins une conduite périphérique (10) comprend au moins une partie verticale.

10. Dispositif de distribution et de collecte selon l'une quelconque des revendications précédentes, dans lequel le canal de collecte (8) comprend une pluralité de points de sortie (13) et le canal de distribution (4) comprend une pluralité de points d'entrée (14), la pluralité de points de sortie (13) étant reliée à la pluralité de points d'entrée (14) par une conduite périphérique (10) unique.

11. Procédé utilisant le dispositif de distribution et de collecte selon l'une quelconque des revendications 1 à 10, comprenant les étapes suivantes :
soutirer un fluide d'un premier lit d'adsorbant (N) par le canal de collecte (8) d'un panneau méridien (12) d'un plateau (n) ;
envoyer le fluide du canal de collecte (8) exclusivement vers une conduite périphérique (10) ; envoyer le fluide de la conduite périphérique (10) exclusivement vers le canal de distribution (4) dudit panneau méridien (12) ;
distribuer le fluide du canal de distribution (4) dans un deuxième lit d'adsorbant (N+1).

12. Procédé selon la revendication 11 pour la séparation des xylènes en lit mobile simulé fonctionnant avec un nombre de lits compris entre 4 et 24.

13. Procédé selon la revendication 11 ou la revendication 12, comprenant en outre l'étape suivante : commander les débits des canaux de distribution (4) du plateau (n) pour alimenter en fluide le deuxième lit d'adsorbant (N+1) avec des vitesses sensiblement égales.

14. Procédé selon l'une quelconque des revendications 11 à 13, dans lequel la vitesse maximale débitante du fluide dans les conduites périphériques (10) est inférieure ou égale à 6 m/s.

15. Procédé selon l'une quelconque des revendications 11 à 14, dans lequel les conduites périphériques (10) fonctionnent à sensiblement iso temps de séjour.

## Patentansprüche

1. Vorrichtung zur Verteilung und Sammlung für eine Einheit zur Trennung im simulierten Bewegtbett mit einem Durchmesser von mehr als 1 Meter, wobei die Vorrichtung mindestens eine Säule umfasst, die in eine Mehrzahl von Adsorberbetten (6) unterteilt ist, und Platten (n), welche die Adsorberbetten (6) tragen, in der:
mindestens eine Platte (n), die zwischen einem ersten Adsorberbett (N) und einem zweiten Adsorberbett (N+1) angeordnet ist, in meridionale Paneele (12) unterteilt ist, die parallel zueinander sind und aneinander angrenzen, um den Querschnitt der Platte (n) abzudecken; und
jedes meridionale Paneel (12) einen Sammelkanal (8), der zum Entnehmen eines Fluids aus dem ersten Adsorberbett (N) geeignet ist, einen Verteilungskanal (4), der zur Verteilung des Fluids zu dem zweiten Adsorberbett (N+1) hin geeignet ist, und eine Trennplatte (11) zum Trennen des Sammelkanals (8) und des Verteilungskanals (4) umfasst,
**dadurch gekennzeichnet, dass** die Vorrichtung eine Mehrzahl von Umfangsleitungen (10) außerhalb der Säule umfasst, wobei jede Umfangsleitung (10) dazu geeignet ist, ausschließlich einen Sammelkanal (8) eines meridionalen Paneels mit dem Verteilungskanal (4) des meridionalen Paneels zu verbinden.

2. Vorrichtung zur Verteilung und Sammlung nach Anspruch 1, bei welcher der Verteilungskanal (4) und der Sammelkanal (8) Höhen haben, die linear über die Länge des meridionalen Paneels (12) variieren, so dass: die Summe der Höhen des Verteilungskanals (4) und des Sammelkanals (8), die an jedem Punkt der Länge des meridionalen Paneels (12) gemessen werden, im Wesentlichen konstant ist; und die Eintrittsgeschwindigkeit von Partikeln oder Molekülen des Fluids in das zweite Adsorberbett (N+1) von der Eintrittsstelle des Verteilungskanals (4) aus über die gesamte Länge des meridionalen Paneels im Wesentlichen konstant bleibt.

3. Vorrichtung zur Verteilung und Sammlung nach Anspruch 1 oder Anspruch 2, bei der die Platte (n) zwischen 2 und 12 meridionale Paneele (12) unterteilt ist.

4. Vorrichtung zur Verteilung und Sammlung nach einem der vorhergehenden Ansprüche, bei der die Umfangsleitungen (10) mindestens eine Injektions- oder Entnahmestelle (9) umfassen, um eine Charge oder ein Lösungsmittel in die Säule zu injizieren oder ein Raffinat oder ein Extrakt aus der Säule zu entnehmen.

5. Vorrichtung zur Verteilung und Sammlung nach einem der vorhergehenden Ansprüche, die eine Mehrzahl von oberen Gittern (7) und unteren Gittern (5) umfasst, wobei die meridionalen Paneele (12) der Platte (n) zwischen dem unteren Gitter (5) des ersten Adsorberbetts (N) und dem oberen Gitter des zweiten Adsorberbetts (N+1) angeordnet sind.

6. Vorrichtung zur Verteilung und Sammlung nach einem der vorhergehenden Ansprüche, bei der die Längen der Umfangsleitungen (10) die an die Platte (n) angeschlossen sind, vorherbestimmt werden, um identische Verweilzeiten der Partikel oder Moleküle des die Umfangsleitungen (10) durchströmenden Fluids zu ermöglichen.

7. Vorrichtung zur Verteilung und Sammlung nach einem der vorhergehenden Ansprüche, bei der jede Umfangsleitung (10) im Wesentlichen dem Perimeter der Säule vom Sammelkanal (8) aus bis zum Verteilungskanal (4) folgt.

8. Vorrichtung zur Verteilung und Sammlung nach einem der vorhergehenden Ansprüche, bei der die Umfangsleitungen (10) im Wesentlichen horizontal sind und am Umfang der Platte (n) gelegen sind, an welche die Umfangsleitungen (10) angeschlossen sind.

9. Vorrichtung zur Verteilung und Sammlung nach einem der vorhergehenden Ansprüche, bei der mindestens eine Umfangsleitung (10) mindestens einen vertikalen Teil umfasst.

10. Vorrichtung zur Verteilung und Sammlung nach einem der vorhergehenden Ansprüche, bei welcher der Sammelkanal (8) eine Mehrzahl von Austrittsstellen (13) umfasst und der Verteilungskanal (4) eine Mehrzahl von Eintrittsstellen (14) umfasst, wobei die Mehrzahl von Austrittsstellen (13) mit der Mehrzahl von Eintrittsstellen (14) durch eine einzige Umfangsleitung (10) verbunden ist.

11. Verfahren, das die Vorrichtung zur Verteilung und Sammlung nach einem der Ansprüche 1 bis 10 verwendet, umfassend die folgenden Schritte:
Entnehmen eines Fluids aus einem ersten Adsorberbett (N) durch den Sammelkanal (8) eines meridionalen Paneels (12) einer Platte (n);
Leiten des Fluids des Sammelkanals (8) ausschließlich zu einer Umfangsleitung (10);
Leiten des Fluids der Umfangsleitung (10) ausschließlich zu dem Verteilungskanal (4) des meridionalen Paneels (12);
Verteilen des Fluids des Verteilungskanals (4) in einem zweiten Adsorberbett (N+1).

12. Verfahren nach Anspruch 11 zur Trennung der Xylole im simulierten Bewegtbett, das mit einer Anzahl von Betten zwischen 4 und 24 funktioniert.

13. Verfahren nach Anspruch 11 oder Anspruch 12, das ferner den folgenden Schritt umfasst: Steuern der Durchsätze der Verteilungskanäle (4) der Platte (n), um das zweite Adsorberbett (N+1) mit im Wesentlichen gleichen Geschwindigkeiten mit Fluid zu versorgen.

14. Verfahren nach einem der Ansprüche 11 bis 13, bei dem die maximale Fördergeschwindigkeit des Fluids in den Umfangsleitungen (10) kleiner als oder gleich 6 m/s ist.

15. Verfahren nach einem der Ansprüche 11 bis 14, bei dem die Umfangsleitungen (10) mit im Wesentlichen gleicher Verweilzeit funktionieren.

## Claims

1. Distribution and collection device for a simulated moving bed separation unit with a diameter of greater than 1 metre, the device comprising at least one column divided into a plurality of adsorbent beds (6) and plates (n) supporting the adsorbent beds (6), in which:
at least one plate (n), positioned between a first adsorbent bed (N) and a second adsorbent bed (N+1), is divided into meridian panels (12) that are mutually parallel and contiguous in order to cover the cross section of said plate (n); and
each meridian panel (12) comprises a collection channel (8) suitable for withdrawing a fluid from the first adsorbent bed (N), a distribution channel (4) suitable for distributing the fluid towards the second adsorbent bed (N+1), and a separation plate (11) to separate the collection channel (8) and the distribution channel (4),
**characterized in that** the device comprises a plurality of peripheral ducts (10) outside of the column, each peripheral duct (10) being suitable for exclusively connecting a collection channel (8) of a meridian panel to the distribution channel (4) of said meridian panel.

2. Distribution and collection device according to Claim 1, in which the distribution channel (4) and the collection channel (8) have heights that vary linearly over the length of the meridian panel (12), such that: the sum of the heights of the distribution channel (4) and of the collection channel (8) taken at any point on the length of the meridian panel (12) is substantially constant; and the inlet velocity into the second adsorbent bed (N+1) of particles or molecules of the fluid remains substantially constant from the inlet point of the distribution channel (4) over the entire length of the meridian panel.

3. Distribution and collection device according to Claim 1 or Claim 2, in which the plate (n) is divided between 2 and 12 meridian panels (12).

4. Distribution and collection device according to any one of the preceding claims, in which the peripheral ducts (10) comprise at least one injection or withdrawal point (9) for injecting a feedstock or a solvent into the column or withdrawing a raffinate or an extract from the column.

5. Distribution and collection device according to any one of the preceding claims, comprising a plurality of upper grids (7) and lower grids (5), the meridian panels (12) of the plate (n) being positioned between the lower grid (5) of the first adsorbent bed (N) and the upper grid of the second adsorbent bed (N+1).

6. Distribution and collection device according to any one of the preceding claims, in which the lengths of the peripheral ducts (10) connected to the plate (n) are predetermined to enable identical residence times of the particles or molecules of the fluid passing through said peripheral ducts (10).

7. Distribution and collection device according to any one of the preceding claims, in which each peripheral duct (10) substantially follows the perimeter of the column from the collection channel (8) to the distribution channel (4).

8. Distribution and collection device according to any one of the preceding claims, in which the peripheral ducts (10) are essentially horizontal and are located at the periphery of the plate (n) to which the peripheral ducts (10) are connected.

9. Distribution and collection device according to any one of the preceding claims, in which at least one peripheral duct (10) comprises at least one vertical portion.

10. Distribution and collection device according to any one of the preceding claims, in which the collection channel (8) comprises a plurality of outlet points (13) and the distribution channel (4) comprises a plurality of inlet points (14), the plurality of outlet points (13) being connected to the plurality of inlet points (14) by a single peripheral duct (10).

11. Process using the distribution and collection device as claimed in any one of Claims 1 to 10, comprising the following steps:
withdrawing a fluid from a first adsorbent bed (N) via the collection channel (8) of a meridian panel (12) of a plate (n);
sending the fluid from the collection channel (8) exclusively to a peripheral duct (10);
sending the fluid from the peripheral duct (10) exclusively to the distribution channel (4) of said meridian panel (12);
distributing the fluid from the distribution channel (4) into a second adsorbent bed (N+1).

12. Process according to Claim 11 for the separation of xylenes in a simulated moving bed operating with a number of beds of between 4 and 24.

13. Process according to Claim 11 or Claim 12, further comprising the following step: controlling the flow rates of the distribution channels (4) of the plate (n) in order to supply fluid to the second adsorbent bed (N+1) with substantially equal velocities.

14. Process according to any one of Claims 11 to 13, in which the maximum discharge velocity of the fluid in the peripheral ducts (10) is less than or equal to 6 m/s.

15. Process according to any one of Claims 11 to 14, in which the peripheral ducts (10) operate with substantially the same residence time (iso-residence time).
